# EUROPEAN PATENT APPLICATION

(11) **EP 2 359 821 A1**
(43) Date of publication of application: **24.08.2011**
(21) Application number: 10179081.4
(22) Date of filing: 28.11.2005
(51) Int. Cl.: A61K 31/135, A61K 31/397, A61P 37/06

(54) **Dosage regimen of an s1p receptor agonist**

(30) Priority: 29.11.2004 US 631483 P
(62) Divisional of application: 05826219.7
(71) Applicant: Novartis AG, 4056 Basel (CH); Novartis Pharma GmbH, 1230 Wien (AT)
(72) Inventor: Kovarik, John M., 4056, Basel (CH); Appel-Dingemanse, Silke, 4123, Allschwil (CH)
(74) Representative: Rouquayrol, Céline Hélène

(57) **Abstract**

S1P receptor modulators or agonists are administered following a dosage regimen whereby during the initial 3 to 6 days of treatment the daily dosage is raised so that in total the R-fold (R being the accumulation factor) standard daily dosage is administered and thereafter continued at the standard daily dosage or at a daily dosage lower than the standard daily dosage.

## Description

The present invention relates to a dosage regimen of an S1P receptor modulator or agonist particularly in the course of the treatment of transplant patients or patients suffering from autoimmune diseases or disorders.

S1P receptor modulators or agonists are compounds which signal as agonists at one or more sphingosine-1 phosphate receptors, e.g. S1P1 to S1P8. Agonist binding to a S1P receptor may e.g. result in dissociation of intracellular heterotrimeric G-proteins into Gα-GTP and Gβγ-GTP, and/or increased phosphorylation of the agonist-occupied receptor and activation of downstream signaling pathways/kinases.

S1P receptor modulators or agonists are valuable compounds for the manufacture of medication for the treatment of various conditions in mammals, especially in human beings. For example, efficacy in transplantation has been demonstrated in rats (skin, heart, liver, small bowel), dogs (kidney), and monkeys (kidney) models. Combination experiments with cyclosporin A showed synergy in skin and heart transplantation models in rats and in monkey renal transplantation. S1P receptor agonists or modulators combined with everolimus prolong survival of cardiac (rat) and renal (monkey) allografts. Due to their immune-modulating potency, S1P receptor modulators or agonists are also useful for the treatment of inflammatory and autoimmune diseases. Further characteristics of S1P receptor agonists can be found in the following publications:
Brinkmann V, Chen S, Feng L, et al (2001) FTY720 alters lymphocyte homing and protects allografts without inducing general immunosuppression. Transplant Proc; 33:530-531.
Brinkmann V, Pinschewer D, Feng L, et al (2001) FTY720: altered lymphocyte traffic results in allograft protection (review). Transplantation; 72:764-769.
Pinschewer DD, Ochsenbein AF, Odermatt B, et al (2000) FTY720 immunosuppression impairs effector T-cell peripheral homing without affecting induction, expansion, and memory. J Immunol; 164:5761.
Yanagawa Y, Sugahara K, Kataoka H, et al (1998) FTY720, a novel immunosuppressant, induces sequestration of circulating mature lymphocytes by acceleration of lymphocyte homing in rats. ll. FTY720 prolongs skin allograft survival by decreasing T cell infiltration into grafts but not cytokine production in vivo. J Immunol.; 160(11):5493-9.

It has now surprisingly been found that a specific dosage regimen, e.g. a loading dose, will provide further unexpected benefits.

The binding affinity of S1P receptor agonists or modulators to individual human S1P receptors may be determined in following assay:
S1P receptor agonist or modulator activities of compounds are tested on the human S1P receptors S1P₁, S1P₂, S1P₃, S1P₄ and S1P₅. Functional receptor activation is assessed by quantifying compound induced GTP [γ-³⁵S] binding to membrane protein prepared from transfected CHO or RH7777 cells stably expressing the appropriate human S1P receptor. The assay technology used is SPA (scintillation proximity based assay). Briefly, DMSO dissolved compounds are serially diluted and added to SPA- bead (Amersham-Pharmacia) immobilised S1P receptor expressing membrane protein (10-20µg/well) in the presence of 50 mM Hepes, 100 mM NaCl, 10 mM MgCl₂, 10 µM GDP, 0.1% fat free BSA and 0.2 nM GTP [γ-³⁵S] (1200 Ci/mmol). After incubation in 96 well microtiterplates at RT for 120 min, unbound GTP [γ-³⁵S] is separated by a centrifugation step. Luminescence of SPA beads triggered by membrane bound GTP [γ-³⁵S] is quantified with a TOPcount plate reader (Packard). EC₅₀S are calculated using standard curve fitting software. In this assay, the S1P receptor modulators or agonists preferably have a binding affinity to S1P receptor <50 nM.

Preferred S1P receptor agonists or modulators are e.g. compounds which in addition to their S1P binding properties also have accelerating lymphocyte homing properties, e.g. compounds which elicit a lymphopenia resulting from a re-distribution, preferably reversible, of lymphocytes from circulation to secondary lymphatic tissue, without evoking a generalized immunosuppression. Naive cells are sequestered; CD4 and CD8 T-cells and B-cells from the blood are stimulated to migrate into lymph nodes (LN) and Peyer's patches (PP).

The lymphocyte homing property may be measured in following Blood Lymphocyte Depletion assay:
A S1P receptor agonist or modulator or the vehicle is administered orally by gavage to rats. Tail blood for hematological monitoring is obtained on day -1 to give the baseline individual values, and at 2, 6, 24, 48 and 72 hours after application. In this assay, the S1P receptor agonist or modulator depletes peripheral blood lymphocytes, e.g. by 50%, when administered at a dose of e.g. < 20 mg/kg.

S1P receptor modulators or agonists are typically sphingosine analogues, such as 2-substituted 2-amino- propane-1,3-diol or 2-amino-propanol derivatives, e. g. a compound comprising a group of formula X 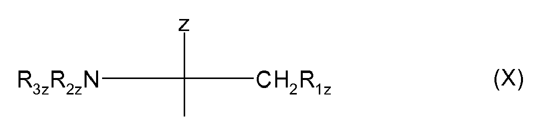 wherein Z is H, C₁₋₆alkyl, C₂₋₆alkenyl, C₂₋₆alkynyl, phenyl, phenyl substituted by OH, C₁₋₆alkyl substituted by 1 to 3 substituents selected from the group consisting of halogen, C₃-₈cycloalkyl, phenyl and phenyl substituted by OH, or CH₂-R_{4z} wherein R_{4z} is OH, acyloxy or a residue of formula (a) 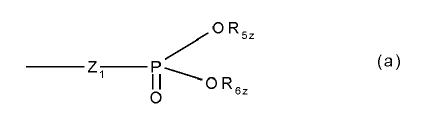
wherein Z₁ is a direct bond or O, preferably O;
each of R_{5z} and R_{6z}, independently, is H, or C₁₋₄alkyl optionally substituted by 1, 2 or 3 halogen atoms;
R_{1z} is OH, acyloxy or a residue of formula (a); and each of R_{2z} and R_{3z} independently, is H, C₁₋₄alkyl or acyl.

Group of formula X is a functional group attached as a terminal group to a moiety which may be hydrophilic or lipophilic and comprise one or more aliphatic, alicyclic, aromatic and/or heterocyclic residues, to the extent that the resulting molecule wherein at least one of Z and R_{1z} is or comprises a residue of formula (a), signals as an agonist at one of more sphingosine-1-phosphate receptor.

Examples of appropriate S1P receptor agonists or modulators are, for example:
- Compounds as disclosed in EP627406A1, e.g. a compound of formula I 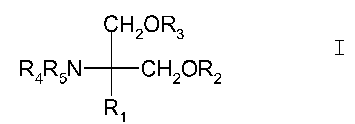 wherein R₁ is a straight- or branched (Cₗ₂-₂₂)chain
   - which may have in the chain a bond or a hetero atom selected from a double bond, a triple bond, O, S, NR₆, wherein R₆ is H, C₁₋₄alkyl, aryl-C₁₋₄alkyl, acyl or (C₁₋₄alkoxy)carbonyl, and carbonyl, and/or
   - which may have as a substituent C₁₋₄alkoxy, C₂₋₄alkenyloxy, C₂₋₄alkynyloxy, arylC₁₋₄alkyl-oxy, acyl, C₁₋₄alkylamino, C₁₋₄alkylthio_{,} acylamino, (C₁₋₄alkoxy)carbonyl, (C₁₋₄alkoxy)-carbonylamino, acyloxy, (C₁₋₄alkyl)carbamoyl, nitro, halogen, amino, hydroxyimino, hydroxy or carboxy; or
      R₁ is
      - a phenylalkyl wherein alkyl is a straight- or branched (C₆₋₂₀)carbon chain; or
      - a phenylalkyl wherein alkyl is a straight- or branched (C₁₋₃₀)carbon chain wherein said phenylalkyl is substituted by
      - a straight- or branched (C₆₋₂₀)carbon chain optionally substituted by halogen,
      - a straight- or branched (C₆₋₂₀)alkoxy chain optionally substitued by halogen,
      - a straight- or branched (C₆₋₂₀)alkenyloxy,
      - phenyl-C₁₋₁₄alkoxy, halophenyl-C₁₋₄alkoxy, phenyl-C₁₋₁₄alkoxy-C₁₋₁₄alkyl, phenoxy-C₁₋₄alkoxy or phenoxy-C₁₋₄alkyl,
      - cycloalkylalkyl substituted by C₆₋₂₀alkyl,
      - heteroarylalkyl substituted by C₆₋₂₀alkyl,
      - heterocyclic C₆₋₂₀alkyl or
      - heterocyclic alkyl substituted by C₂₋₂₀alkyl,
         and wherein
         the alkyl moiety may have
      - in the carbon chain, a bond or a heteroatom selected from a double bond, a triple bond, O, S, sulfinyl, sulfonyl, or NR₆, wherein R₆ is as defined above, and
      - as a substituent C₁₋₄alkoxy, C₂₋₄alkenyloxy, C₂₋₄alkynyloxy, arylC₁₋₄alkyloxy, acyl, C₁₋₄alkylamino, C₁₋₄alkylthio, acylamino, (C₁₋₄alkoxy)carbonyl, (C₁₋₄alkoxy)carbonylamino, acyloxy, (C₁₋₄alkyl)carbamoyl, nitro, halogen, amino, hydroxy or carboxy, and
         each of R₂, R₃, R₄ and R₅, independently, is H, C₁₋₄ alkyl or acyl
         or a pharmaceutically acceptable salt or hydrate thereof;
- Compounds as disclosed in EP 1002792A1, e.g. a compound of formula II 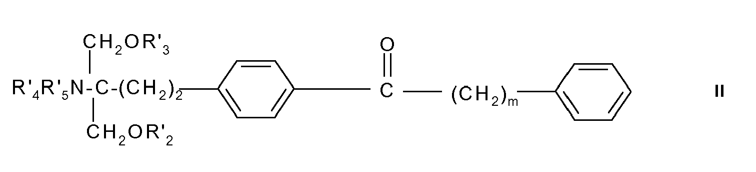 wherein m is 1 to 9 and each of R'₂, R'₃, R'₄ and R'₅, independently, is H, C₁₋₆alkyl or acyl, or a pharmaceutically acceptable salt or hydrate thereof;
- Compounds as disclosed in EP0778263 A1, e.g. a compound of formula III 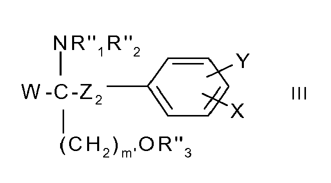 wherein W is H; C₁₋₆alkyl, C₂₋₆alkenyl or C₂₋₆alkynyl; unsubstituted or by OH substituted phenyl; R"₄O(CH₂)ₙ; or C₁₋₆alkyl substituted by 1 to 3 substituents selected from the group consisting of halogen, C₃₋₈Cycloalkyl, phenyl and phenyl substituted by OH;
   X is H or unsubstituted or substituted straight chain alkyl having a number p of carbon atoms or unsubstituted or substituted straight chain alkoxy having a number (p-1) of carbon atoms, e.g. substituted by 1 to 3 substitutents selected from the group consisting of C₁₋₆alkyl, OH, C₁-₆alkoxy, acyloxy, amino, C₁₋₆alkylamino, acylamino, oxo, haloC₁₋₆alkyl, halogen, unsubstituted phenyl and phenyl substituted by 1 to 3 substituents selected from the group consisting of C₁-₆alkyl, OH, C₁₋₆alkoxy, acyl, acyloxy, amino, C₁₋₆alkylamino, acylamino, haloC₁₋₆alkyl and halogen; Y is H, C₁₋₆alkyl, OH, C₁₋₆alkoxy, acyl, acyloxy, amino, C₁₋₆alkylamino, acylamino, haloC₁₋₆alkyl or halogen, Z₂ is a single bond or a straight chain alkylene having a number or carbon atoms of q,
   each of p and q, independently, is an integer of 1 to 20, with the proviso of 6≤p+q≤23, m' is 1, 2 or 3, n is 2 or 3,
   each of R"₁, R"₂, R"₃ and R"₄, independently, is H, C₁₋₄alkyl or acyl,
   or a pharmaceutically acceptable salt or hydrate thereof,
- Compounds as disclosed in WO02/18395, e.g. a compound of formula IVa or IVb 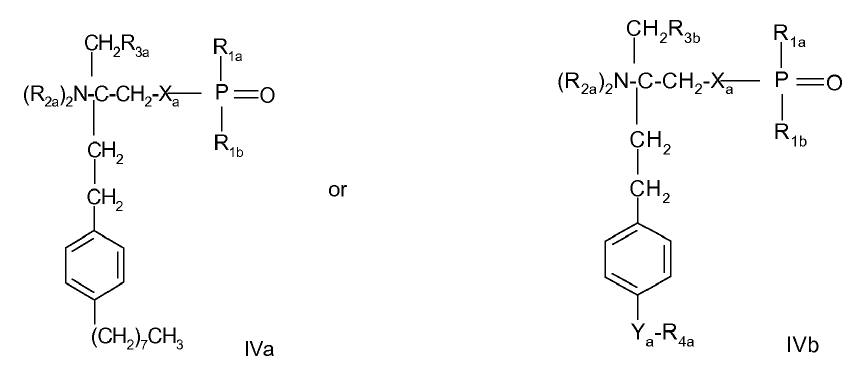 wherein Xₐ is O, S, NR₁ₛ or a group -(CH₂)ₙₐ-, which group is unsubstituted or substituted by 1 to 4 halogen; nₐ is 1 or 2, R₁ₛ is H or (C₁₋₄)alkyl, which alkyl is unsubstituted or substituted by halogen; R₁ₐ is H, OH, (C₁₋₄)alkyl or O(C₁₋₄)alkyl wherein alkyl is unsubstituted or substituted by 1 to 3 halogen; R_{1b} is H, OH or (C₁₋₄)alkyl, wherein alkyl is unsubstituted or substituted by halogen; each R₂ₐ is independently selected from H or (C₁₋₄)alkyl, which alkyl is unsubstituted or substitued by halogen; R₃ₐ is H, OH, halogen or O(C₁₋₄)alkyl wherein alkyl is unsubstituted or substituted by halogen; and R_{3b} is H, OH, halogen, (C₁₋₄)alkyl wherein alkyl is unsubstituted or substituted by hydroxy, or O(C₁₋₄)alkyl wherein alkyl is unsubstituted or substituted by halogen; Yₐ is -CH₂-, -C(O)-, -CH(OH)-, -C(=NOH)-, O or S, and R₄ₐ is (C₄₋₁₄)alkyl or (C₄₋₁₄)alkenyl;
   or a pharmaceutically acceptable salt or hydrate thereof;
- Compounds as disclosed in WO 02/076995, e.g. a compound of formula V 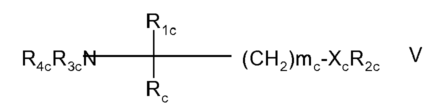 wherein
   - m_{c}: is 1, 2 or 3;
   - X_{c}: is O or a direct bond;
   - R_{1c}: is H; C₁₋₆ alkyl optionally substituted by OH, acyl, halogen, C₃₋₁₀cycloalkyl, phenyl or hydroxy-phenylene; C₂₋₆alkenyl; C₂₋₆alkynyl; or phenyl optionally substituted by OH;
   - R_{2c}: is 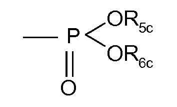 wherein R_{5c} is H or C₁₋₄alkyl optionally substituted by 1, 2 or 3 halogen atoms, and R_{6c} is H or C₁₋₄alkyl optionally substituted by halogen;
   each of R_{3c} and R_{4c}, independently, is H, C₁-₄alkyl optionally substituted by halogen, or acyl, and
   - R_{c}: is C₁₃₋₂₀alkyl which may optionally have in the chain an oxygen atom and which may optionally be substituted by nitro, halogen, amino, hydroxy or carboxy; or a residue of formula (a) 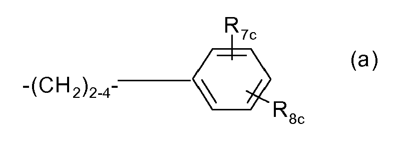 wherein R_{7c} is H, C₁₋₄alkyl or C₁₋₄alkoxy, and R_{8c} is substituted C₁₋₂₀alkanoyl, phenylC₁₋₁₄alkyl wherein the C₁₋₁₄alkyl is optionally substituted by halogen or OH, cycloalkylC₁₋₁₄alkoxy or phenylC₁₋₁₄alkoxy wherein the cycloalkyl or phenyl ring is optionally substituted by halogen, C₁₋₄alkyl and/or C₁₋₄alkoxy, phenylC₁₋₁₄alkoxy-C₁₋₁₄alkyl, phenoxyC₁₋₁₄alkoxy or phenoxyC₁₋₁₄alkyl,
   - R_{c}: being also a residue of formula (a) wherein R_{8c} is C₁₋₁₄alkoxy when R_{1c} is C₁₋₄alkyl, C₂₋₆alkenyl or C₂₋₆salkynyl,
   or a compound of formula Vl 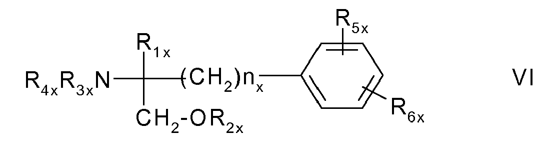 wherein
   nₓ is 2, 3 or 4
   R₁ₓ is H; C₁₋₆alkyl optionally substituted by OH, acyl, halogen, cycloalkyl, phenyl or hydroxy-phenylene; C₂₋₆alkenyl; C₂₋₆alkynyl; or phenyl optionally substituted by OH;
   R₂ₓ is H, C₁₋₄ alkyl or acyl
   each of R₃ₓ and R₄ₓ, independently is H, C₁₋₄alkyl optionally substituted by halogen or acyl,
   R₅ₓ is H, C₁₋₄alkyl or C₁₋₄alkoxy, and
   R₆ₓ is C₁₋₂₀ alkanoyl substituted by cycloalkyl; cyloalkylC₁₋₁₄alkoxy wherein the cycloalkyl ring is optionally substituted by halogen, C₁₋₄alkyl and/or C₁₋₄alkoxy; phenylC₁₋₁₄alkoxy wherein the phenyl ring is optionally substituted by halogen, C₁₋₄alkyl and/or C₁₋₄alkoxy,
   R₆ₓ being also C₄₋₁₄alkoxy when R₁ₓ is C₂₋₄alkyl substituted by OH, or pentyloxy or hexyloxy when R₁ₓ is C₁₋₄akyl,
   provided that R₆ₓ is other than phenyl-butylenoxy when either R₅ₓ is H or R₁ₓ is methyl,
   or a pharmaceutically acceptable salt or hydrate thereof;
- Compounds as disclosed in WO002/06268AI, e.g. a compound of formula VII 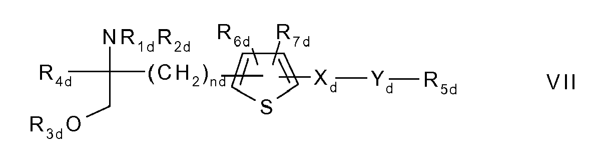
   wherein each of R_{1d} and R_{2d}, independently, is H or an amino-protecting group;
   R_{3d} is hydrogen, a hydroxy-protecting group or a residue of formula 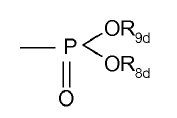
      R_{4d} is C₁₋₄alkyl;
      n_{d} is an integer of 1 to 6;
      X_{d} is ethylene, vinylene, ethynylene, a group having a formula - D-CH₂- (wherein D is carbonyl, - CH(OH)-, O, S or N), aryl or aryl substituted by up to three substitutents selected from group a as defined hereinafter;
      Y_{d} is single bond, C₁₋₁₀alkylene, C₁₋₁₀alkylene which is substituted by up to three substitutents selected from groups a and b, C₁₋₁₀alkylene having O or S in the middle or end of the carbon chain, or C₁₋₁₀alkylene having O or S in the middle or end of the carbon chain which is substituted by up to three substituents selected from groups a and b;
      R_{5d} is hydrogen, C₃₋₆cycloalkyl, aryl, heterocyclic group, C₃₋₆cycloalkyl substituted by up to three substituents selected from groups a and b, aryl substituted by up to three substituents selected from groups a and b, or heterocyclic group substituted by up to three substituents selected from groups a and b;
      each of R_{6d} and R_{7d}, independently, is H or a substituent selected from group a;
      each of R_{8d} and R_{9d}, independently, is H or C₁₋₄alkyl optionally substituted by halogen;
      <group a > is halogen, lower alkyl, halogeno lower alkyl, lower alkoxy, lower alkylthio, carboxyl, lower alkoxycarbonyl, hydroxy, lower aliphatic acyl, amino, mono-lower alkylamino, di-C₁₋₄alkylamino, acylamino, cyano or nitro; and
      <group b > is C₃₋₆cycloalkyl, aryl or heterocyclic group, each being optionally substituted by up to three substituents selected from group a;
      with the proviso that when R_{5d} is hydrogen, Y_{d} is a either a single bond or linear C₁₋₁₀ alkylene, or a pharmacologically acceptable salt, ester or hydrate thereof;
- Compounds as disclosed in JP-14316985 (JP2002316985), e.g. a compound of formula VIII 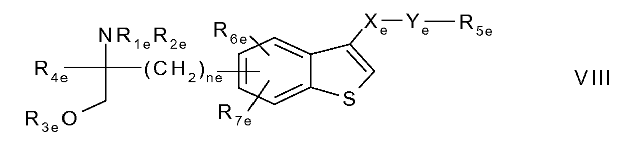
   wherein R₁ₑ,R₂ₑ,R₃ₑ,R₄ₑ,R₅ₑ,R₆ₑ,R₇ₑ, nₑ, Xₑ and Yₑ are as disclosed in JP-14316985;
   or a pharmacologically acceptable salt, ester or hydrate thereof;
- Compounds as disclosed in WO 03/29184 and WO 03/29205, e.g. compounds of formula lX 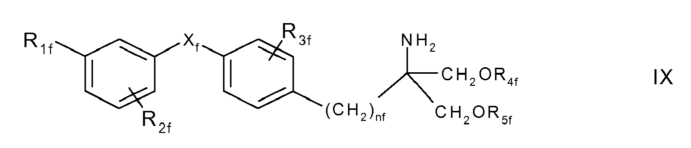
   wherein X_{f} is O, S, SO or SO₂
   R_{1f} is halogen, trihalomethyl, OH, C₁₋₇alkyl, C₁₋₄alkoxy, trifluoromethoxy, phenoxy, cyclohexylmethyloxy, pyridylmethoxy, cinnamyloxy, naphthylmethoxy, phenoxymethyl, CH₂-OH, CH₂-CH₂-OH, C₁₋₄alkylthio, C₁₋₄alkylsulfinyl, C₁₋₄alkylsulfonyl, benzylthio, acetyl, nitro or cyano, or phenyl, phenylC₁₋₄alkyl or phenyl-C₁₋₄alkoxy each phenyl group thereof being optionally substituted by halogen, CF₃, C₁₋₄alkyl or C₁₋₄alkoxy;
   R_{2f} is H, halogen, trihalomethyl, C₁₋₄alkoxy, C₁₋₇alkyl, phenethyl or benzyloxy;
   R_{3f} H, halogen, CF₃, OH, C₁₋₇alkyl, C₁₋₄alkoxy, benzyloxy or C₁₋₄alkoxymethyl;
   each of R₄fand R_{5f}, independently is H or a residue of formula 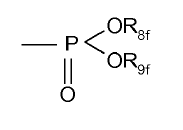
      wherein each of R_{8f} and R_{9f}, independently, is H or C₁₋₄alkyl optionally substituted by halogen;and
      n_{f} is an integer from 1 to 4;
      e.g. 2-amino-2-[4-(3-benzyloxyphenoxy)-2-chlorophenyl]ethyl-1,3-propane-diol, 2-amino-2-[4-(benzyloxyphenylthio)-2- chlorophenyl]ethyl-1,3-propane-diol, 2-amino-2-[4-(3-benzyloxyphenoxy)-2-chlorophenyl]propyl-1,3-propane-diol or 2-amino-2-[4-(benzyloxyphenylthio)-2- chlorophenyl]propyl-1,3-propane-diol,
      or a pharmacological salt, solvate or hydrate thereof;
- Compounds as disclosed in WO03/062252A1, e.g. a compound of formula X 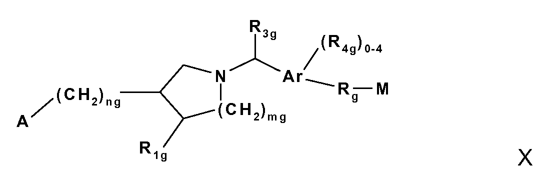 wherein
   Ar is phenyl or naphthyl; each of mg and ng independently is 0 or 1; A is selected from COOH, PO₃H₂, PO₂H,SO₃H PO(C₁₋₃alkyl)OH and 1 H-tetrazol-5-yl; each of R₁g and R₂g independently is H, halogen, OH, COOH or C₁₋₄alkyl optionally substituted by halogen; R₃g is H or C₁₋₄alkyl optionally substituted by halogen or OH; each R₄g independently is halogen, or optionally halogen substituted C₁₋₄alkyl or C₁₋₃alkoxy; and each of Rg and M has one of the significances as indicated for B and C, respectively, in WO03/062252A1;
   or a pharmacologically acceptable salt, solvate or hydrate thereof;
- Compounds as disclosed in WO 03/062248A2, e.g. a compound of formula Xl 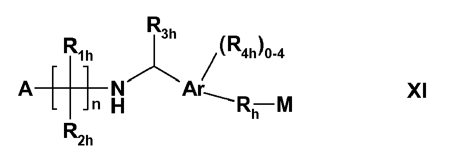 wherein Ar is phenyl or naphthyl; n is 2,3 or 4; A is COOH, 1*H*-tetrazol-5-yl, PO₃H₂, PO₂H₂, - SO₃H or PO(R₅ₕ)OH wherein R₅ₕ is selected from C₁₋₄alkyl, hydroxyC₁₋₄alkyl, phenyl, -CO-C₁₋₃alkoxy and -CH(OH)-phenyl wherein said phenyl or phenyl moiety is opitonally substituted; each of R₁ₕ and R₂ₕ independently is H, halogen, OH, COOH, or optionally halogeno substituted C₁₋₆alkyl or phenyl; R₃ₕ is H or C₁₋₄alkyl optionally substituted by halogen and/ OH; each R₄ₕ independently is halogeno, OH, COOH, C₁₋₄alkyl, S(O)₀,_{1 or2}C₁₋₃alkyl, C₁-₃alkoxy, C₃₋₆Cycloalkoxy, aryl or aralkoxy, wherein the alkyl portions may optionally be substituted by 1-3 halogens; and each of Rₕ and M has one of the significances as indicated for B and C, respectively, in WO03/062248A2;
   or a pharmacologically acceptable salt, solvate or hydrate thereof;
- Compounds as disclosed in WO 04/103306A, WO 05/000833, WO 05/103309 or WO 05/113330, e.g. compounds of formula Xlla or Xllb 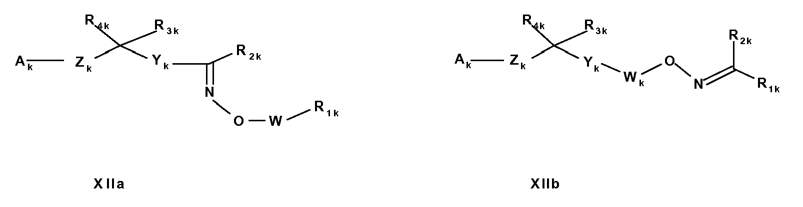 wherein
   Aₖ is COOR₅ₖ, OPO(OR₅ₖ)₂, PO(OR₅ₖ)₂, SO₂OR₅ₖ, POR₅ₖOR₅ₖ or 1 H-tetrazol-5-yl, R₅ₖ being H or C₁₋₆alkyl;
   Wₖ is a bond, C₁₋₃alkylene or C₂₋₃alkenylene;
   Yₖ is C₆₋₁₀aryl or C₃₋₉heteroaryl, optionally substituted by 1 to 3 radicals selected from halogene, OH, NO₂, C₁₋₆alkyl, C₁₋₆alkoxy; halo-substituted C₁₋₆alkyl and halo-substituted C₁₋₆alkoxy;
   Zₖ is a heterocyclic group as indicated in WO 04/103306A, e.g. azetidine;
   R₁ₖ is C₆₋₁₀aryl or C₃₋₉heteroaryl, optionally substituted by C₁₋₆alkyl, C₆₋₁₀aryl, C₆₋₁₀arylC₁₋₄alkyl, C₃₋₉heteroaryl, C₃₋₉heteroarylC₁₋₄alkyl, C₃₋₈cydoalkyl, C₃₋₈cycloalkylC₁₋₄alkyl, C₃₋₈heterocycloalkyl or C₃₋₈heterocycloalkylC₁₋₄alkyl; wherein any aryl, heteroaryl, cycloalkyl or heterocycloalkyl of R₁ₖ may be substituted by 1 to 5 groups selected from halogen, C₁₋₆alkyl, C₁₋₆alkoxy and halo substituted-C₁₋6alkyl or -C₁₋₆alkoxy;
   R₂ₖ is H, C₁₋₆alkyl, halo substituted C₁₋₆alkyl, C₂₋₆alkenyl or C₂₋₆alkynyl: and
   each of R₃ₖ or R₄ₖ, independently, is H, halogen, OH, C₁₋₆alkyl, C₁₋₆alkoxy or halo substituted C₁₋₆alkyl or C₁₋₆alkoxy;
   and the N-oxide derivatives thereof or prodrugs thereof,
   or a pharmacologically acceptable salt, solvate or hydrate thereof.

According to a further embodiment of the invention, a S1P receptor agonist or modulator for use in a combination of the invention may also be a selective S1P1 receptor, e.g. a compound which possesses a selectivity for the S1P1 receptor over the S1P3 receptor of at least 20 fold, e.g. 100, 500, 1000 or 2000 fold, as measured by the ratio of EC₅₀ for the S1P1 receptor to the EC₅₀ for the S1P3 receptor as evaluated in a ³⁵S-GTPγS binding assay, said compound having an EC₅₀ for binding to the S1P1 receptor of 100 nM or less as evaluated by the ³⁵S-GTPγS binding assay. Representative S1P1 receptor agonists or modulators are e.g. the compounds listed in WO 03/061567, the contents of which being incorporated herein by reference, for instance a compound of formula XIII or XIV

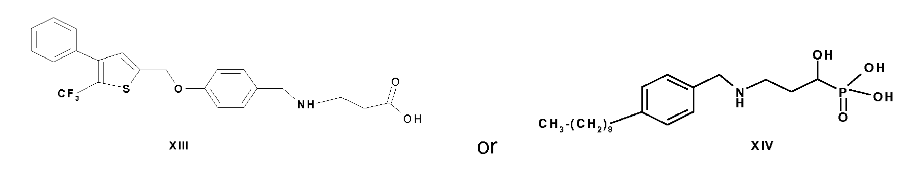

When the compounds of formulae I to XIV have one or more asymmetric centers in the molecule, the present invention is to be understood as embracing the various optical isomers, as well as racemates, diastereoisomers and mixtures thereof are embraced. Compounds of formula III or IVb, when the carbon atom bearing the amino group is asymmetric, have preferably the R-configuration at this carbon atom.

The compounds of formulae I to XIV may exist in free or salt form. Examples of pharmaceutically acceptable salts of the compounds of the formulae I to XIV include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals such as sodium, potassium, calcium and aluminium, salts with amines, such as triethylamine and salts with dibasic amino acids, such as lysine. The compounds and salts of the combination of the present invention encompass hydrate and solvate forms.

Acyl as indicated above may be a residue R_{y}-CO- wherein R_{y} is C₁₋₆alkyl, C₃₋₆cycloalkyl, phenyl or phenyl-C₁₋₄alkyl. Unless otherwise stated, alkyl, alkoxy, alkenyl or alkynyl may be straight or branched.

Aryl may be phenyl or naphthyl, preferably phenyl.

When in the compounds of formula I the carbon chain as R₁ is substituted, it is preferably substituted by halogen, nitro, amino, hydroxy or carboxy. When the carbon chain is interrupted by an optionally substituted phenylene, the carbon chain is preferably unsubstituted. When the phenylene moiety is substituted, it is preferably substituted by halogen, nitro, amino, methoxy, hydroxy or carboxy.

Preferred compounds of formula I are those wherein R₁ is C₁₃₋₂₀alkyl, optionally substituted by nitro, halogen, amino, hydroxy or carboxy, and, more preferably those wherein R₁ is phenylalkyl substituted by C₆₋₁₄-alkyl chain optionally substituted by halogen and the alkyl moiety is a C₁₋₆alkyl optionally substituted by hydroxy. More preferably, R₁ is phenyl-C₁₋₆alkyl substituted on the phenyl by a straight or branched, preferably straight, C₆₋₁₄alkyl chain. The C₆₋₁₄alkyl chain may be in ortho, meta or para, preferably in para.

Preferably each of R₂ to R₅ is H.

In the above formula of VII "heterocyclic group" represents a 5- to 7 membered heterocyclic group having 1 to 3 heteroatoms selected from S, O and N. Examples of such heterocyclic groups include the heteroaryl groups indicated above, and heterocyclic compounds corresponding to partially or completely hydrogenated heteroaryl groups, e.g. furyl, thienyl, pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyranyl, pyridyl, pyridazinyl, pyrimidinyl, pyrazinyl, tetrahydropyranyl, morpholinyl, thiomorpholinyl, pyrrolidinyl, pyrrolyl, imidazolidinyl, pyrazolidinyl, piperidinyl, piperazinyl, oxazolidinyl, isoxazolidinyl, thiazolidinyl or pyrazolidinyl. Preferred heterocyclic groups are 5-or 6-membered heteroaryl groups and the most preferred heteocyclic group is a morpholinyl, thiomorpholinyl or piperidinyl group.

A preferred compound of formula I is 2-amino-2-tetradecyl-1,3-propanediol. A particularly preferred S1P receptor agonist of formula I is FTY720, i.e. 2-amino-2-[2-(4-octylphenyl) ethyl]propane-1 ,3-diol in free form or in a pharmaceutically acceptable salt form (referred to hereinafter as Compound A), e.g. the hydrochloride, as shown:

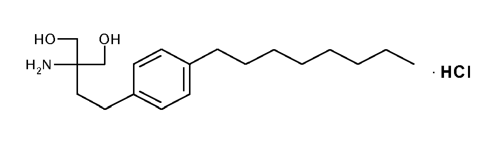

A preferred compound of formula II is the one wherein each of R'₂ to R'₅ is H and m is 4, i.e. 2-amino-2-{2-[4-(1-oxo-5-phenylpentyl)phenyl]ethyl}propane-1,3-diol, in free form or in pharmaceutically acceptable salt form (referred to hereinafter as Compound B), e.g the hydrochloride.

A preferred compound of formula III is the one wherein W is CH₃, each of R"₁ to R"₃ is H, Z₂ is ethylene, X is heptyloxy and Y is H, i.e. 2-amino-4-(4-heptyloxyphenyl)-2-methyl-butanol, in free form or in pharmaceutically acceptable salt form (referred to hereinafter as Compound C), e.g. the hydrochloride. The R-enantiomer is particularly preferred.

A preferred compound of formula IVa is the FTY720-phosphate (R₂ₐ is H, R₃ₐ is OH, Xₐ is O, R₁ₐ and R_{1b} are OH). A preferred compound of formula IVb is the Compound C-phosphate (R₂ₐ is H, R_{3b} is OH, Xₐ is O, R₁ₐ and R_{1b} are OH, Yₐ is O and R₄ₐ is heptyl). A preferred compound of formula V is Compound B-phosphate.

A preferred compound of formula V is phosphoric acid mono-[(R)-2-amino-2-methyl-4-(4-pentyloxy-phenyl)-butyl]ester.

A preferred compound of formula VIII is (2R)-2-amino-4-[3-(4-cyclohexyloxybutyl)-benzo[b]thien-6-yl]-2-methylbutan-1-ol.

A preferred compound of formula Xlla is e.g. 1-{4-[1-(4-cyclohexyl-3-trifluoromethyl-benzyloxyimino)-ethyl]-2-ethyl-benzyl}-azetidine-3-carboxylic acid, or a prodrug thereof.

According to the invention, it provides the use of an S1P receptor modulator or agonist in the manufacture of a medication, whereby said medication is administered in such a way that during the initial 3 to 6 days, preferably 4 or 5 days, most preferred 4 days, of treatment the dosage of said S1P receptor modulator or agonist is raised so that in total the R-fold (R being the accumulation factor) standard daily dosage of said S1P receptor modulator or agonist is administered and thereafter the treatment is continued with the standard or a lower daily dosage of said S1P receptor modulator or agonist.

Preferred medications comprise medication for transplant patients providing prolonged survival rates, in particular prolonged allograft survival rates especially for renal, heart, lung or liver transplants, or for patients suffering from autoimmune diseases, e.g. multiple sclerosis, lupus nephritis, rheumatoid arthritis, inflammatory bowel diseases or psoriasis.

In view of the normally prolonged taking of the medication, the standard daily dosage (also called maintenance dose) refers to the dosage of an S1P receptor modulator or agonist necessary for a steady-state trough blood level of the medication or its active metabolite(s) providing effective treatment. Said dosage is dependent on the accumulation factor (R). By blood level is meant the concentration of a drug in blood at any time. Trough blood level corresponds to a pre-dose blood level. Steady-state means whether the trough or blood level is stable over time. Steady-state trough blood levels may be assessed, for example, by obtaining a pre-dose blood sample anytime after month 3. The accumulation factor (R) is calculated on the ratio of the steady-state trough to the trough just before the second dose.

Preferably, the dosage of the S1P receptor modulator or agonist during the initial 3 to 6 days, of treatment is increased stepwise. Thereafter the treatment is continued with the maintenance therapy with the standard daily dosage or with a lower daily dosage. When the treatment is continued at a lower daily dosage, it may be e.g. about 1/50 to ½, preferably 1/50 to 1/10, of the standard daily dosage of the S1P receptor modulator or agonist.

Preferably, the total dosage of said S1P receptor modulator or agonist during the initial 3 to 6 days, preferably 4 or 5 days, most preferred 4 days, of treatment is increased incrementally from 3- to 21-fold, more preferred from 4 to 12-fold, particularly about 10-fold, the standard daily dosage of said S1P receptor modulator or agonist. For example, the loading dose may be 1; 1.5-2; 2-3; and 3-4 fold the standard daily dosage, on day 1, 2, 3 and 4, respectively.

According to a preferred embodiment of the invention, the highest loading regimen dose instalment on the last day of the loading regimen, e.g. on day 4, is 4x the maintenance dose of the S1P receptor modulator or agonist. The instalment doses on days 1, 2 and 3 of the loading regimen may be e.g. about ¼ ; ½; and ¾ of the highest instalment dose of the S1P receptor modulator or agonist.

A particularly preferred dosage of the S1P receptor modulator or agonist, e.g. the preferred S1P receptor modulator FTY720, is e.g. 2-5, 5-10, 10-15 and 15-20 mg, e.g. a regimen of 2.5mg/5mg/7.5mg/10mg or 5mg/10mg/15mg/20mg, respectively, during the initial period of 4 days. Thereafter the treatment is continued with the maintenance therapy, e.g. a daily dosage of 2.5 mg or 5 mg, or at a lower daily dosage, e.g. 0.1 to 0,5 mg.

In a further embodiment of the invention, a preferred loading regimen of a S1P receptor agonist or modulator, e.g. the preferred S1P receptor modulator FTY720, may also be e.g. 0.5mg/1 mg/1.5mg/2mg during the initial period of 4 days. Thereafter the treatment is continued with the maintenance therapy, e.g. a daily dosage of 0,5 mg.

In a series of further specific or alternative embodiments, the present invention also provides:
1.1 The use of a S1P receptor modulator or agonist, e.g. FTY720, in the manufacture of a medication, whereby said medication is administered in such a way to a subject that a steady-state of the S1P receptor modulator or agonist blood levels is attained in less than a week.
   The steady-state attained is such that the subject is sufficiently immunosuppressed, e.g. it shows no signs or symptoms of acute graft rejection or relapse or rebound of the autoimmune disease. During the initial 3 to 6 days, preferably 4 or 5 days, most preferred 4 days, of treatment the daily dosage of the S1P receptor modulator or agonist is raised stepwise up to 3- to 21-fold the standard daily dosage of said S1P receptor modulator or agonist and thereafter the treatment is continued with the standard daily dosage of said S1P receptor modulator or agonist.
1.2 The use of a S1P receptor modulator or agonist, e.g. FTY720, in the manufacture of a medication, whereby said medication is administered in such a way to a subject that a steady-state of the S1P receptor modulator or agonist blood levels is attained in less than a week, and thereafter the treatment is continued at a dosage lower than the standard daily dosage.
1.3. The use of a S1P receptor modulator or agonist, e.g. FTY720, in the manufacture of a medication, whereby said medication is administered in such a way that during the initial 4 days of treatment the dosage of the S1P receptor modulator or agonist is 1; 1.5-2; 2-3; and 3-4 fold the standard daily dosage, respectively, and thereafter the treatment is continued with the standard daily dosage of the S1P receptor modulator or agonist, or at a lower daily dosage.
1.4 The use of a S1P receptor modulator or agonist, e.g. FTY720, in the manufacture of a medication, whereby said medication is administered in such a way that during the initial 4 days of treatment the dosage of the S1P receptor modulator or agonist is ¼ ½; and ¾ of the highest instalment dose of the S1P receptor modulator or agonist; and 4x the maintenance dose of the S1P receptor modulator or agonist, respectively, and thereafter the treatment is continued with the maintenance dose or optionally with a lower daily dosage of the S1P receptor modulator or agonist.
1.5 The use of an S1P receptor modulator or agonist, e.g. FTY720, in the manufacture of a medication, whereby said medication is administered in such a way that during the initial 3 to 6 days, preferably 4 or 5 days, most preferred 4 days, of treatment the dosage of said S1P receptor modulator or agonist is raised so that in total the R-fold standard daily dosage of said S1P receptor modulator or agonist is administered and thereafter the treatment is continued with the standard daily dosage of said S1P receptor agonist or at a lower daily dosage.
1.6 The use of an S1P receptor modulator or agonist, e.g. FTY720, in the manufacture of a medication, whereby said medication is administered, after a loading regimen, at a daily dosage which is lower than the standard daily dosage.
1.7 The use of FTY720 in the manufacture of a medication, whereby said medication is administered, after a loading regimen, at a daily dosage of 0.1 to 0.5 mg.
2. A method for inhibiting graft rejection or treating an autoimmune disease in a subject in need thereof, comprising administering to the subject a S1P receptor modulator or agonist, e.g. FTY720, in such a pharmaceutically effective amount that a steady-state of the S1P receptor modulator or agonist blood levels is attained in the subject in less than a week. Thereafter the treatment is continued with the standard daily dosage of said S1P receptor modulator or agonist or at a lower daily dosage
2.1 A method for producing a steady-state of S1P receptor modulator or agonist blood levels in a subject in less than a week comprising administering during the initial 3 to 6 days, preferably 4 or 5 days, most preferred 4 days, an incremental daily dosage of up 3- to 21-fold the standard daily dosage of said S1P receptor modulator or agonist.
2.2 In a treatment method with a S1P receptor modulator or agonist, e.g. FTY720, the improvement being that the S1P receptor modulator or agonist is administered in such a way that during the initial 3 to 6 days, preferably 4 or 5 days, most preferred 4 days, of treatment the dosage is raised so that in total the R-fold standard daily dosage is administered. Thereafter the treatment is continued with the standard effective daily dosage or at a lower daily dosage.
2.3 A method for providing prolonged transplant survival rates in a subject, whereby an S1P receptor modulator or agonist is administered in such a way that during the initial 3 to 6 days, preferably 4 or 5 days, most preferred 4 days, of treatment the dosage is raised stepwise so that in total the R-fold standard daily dosage is administered and thereafter the treatment is continued with the standard daily dosage or at a lower daily dosage.
2.4 A method for inhibiting graft rejection or treating an autoimmune disease in a subject in need thereof, comprising administering to the subject, after a loading regimen, a S1 P receptor modulator or agonist, e.g. FTY720, at a daily dosage which is lower than the standard daily dosage.
2.5 A method for treating an autoimmune disease in a subject in need thereof, comprising administering to the subject, after a loading regimen, a daily dosage of FTY720 of about 0.1 to 0.5mg.
3. A kit containing daily units of medication of an S1P receptor modulator or agonist, e.g. FTY720, of varying daily dosage, whereby the daily dosage of said S1P receptor modulator or agonist for the initial 3 to 6 days, preferably 4 or 5 days, most preferred 4 days, of treatment is incrementally increased so that the total amount present in the daily units corresponds to the R-fold standard daily dosage of said S1P receptor modulator or agonist for this initial time period.
3.1. A kit containing daily units of medication of an S1P receptor modulator or agonist, e.g. FTY720, of varying daily dosage, whereby the daily dosage of S1P receptor modulator or agonist for the initial 4 days of treatment is 1; 1.5-2; 2-3; and 3-4 fold the standard daily dosage, respectively. The kit may further comprise units for the standard daily dosage of the S1P receptor modulator or agonist, e.g. FTY720, or for the subsequent treatment with a lower daily dosage. The kit may also contain instructions for use.
3.2 A kit containing daily units of medication of an S1P receptor modulator or agonist, e.g. FTY720, of varying daily dosage, whereby the daily dosage of S1P receptor modulator or agonist for the initial 4 days of treatment is ¼; ½; and ¾ of the highest instalment dose of the S1P receptor modulator or agonist; and 4x the maintenance dose of the S1P receptor modulator or agonist, respectively. The kit may further comprise units for the standard daily dosage of the S1P receptor modulator or agonist, e.g. FTY720, or for the subsequent treatment with a lower daily dosage. The kit may also contain instructions for use.

The loading regimen of S1P receptor modulator or agonist which is administered to the subject according to the invention may be given either during the initial 3-6 days post-transplantation or may start even prior to the transplantation surgery, or at the beginning of an autoimmune disease therapy, or after an interruption of S1P receptor modulator or agonist therapy.

Utility of an S1P receptor modulator or agonist dosage regimen in treating diseases and conditions as hereinabove specified may be demonstrated in standard animal or clinical tests, e.g. in accordance with the methods described hereinafter.

### 2-Phase Loading Regimen-Study:

Initial baseline (Day -2): on Day -2, subjects enter the study center at least 12-hours prior to dosing for verification of inclusion/exclusion criteria and baseline assessments.

Placebo run-in (Day -1): On Day -1, subjects receive a single, placebo dose of FTY720

FTY720 treatment (Days 1-7): All subjects receive FTY720 once daily for 7 consecutive days as follows,
Day 1: Subjects receive a single 5 mg FTY720 oral dose at the exact time the Day -1 dose was administered.
Days 2-4: Subject receive a single 10 mg FTY720 oral dose on Day 2, a single 15 mg FTY720 oral dose on Day 3, and a single 20 mg FTY720 oral dose on Day 4, in order to achieve the FTY720 steady-state concentration typically measured in patients on chronic dosing of FTY720 5 mg qd.
Day 5-7: Subjects receive single 5 mg FTY720 oral doses once daily.

Pharmacokinetic, pharmacodynamic and safety assessments are performed at specified times during the multiple-dose study. Subjects are released from the study center approximately 24 hours after the last drug administration on Day 7, after the safety evaluations have been completed (i.e., Day 8).
- Analytes, media and methods:
   FTY720 is measured in whole blood using LC/MS/MS (LLOQ = 0.080 ng/mL)
- PK evaluations: Noncompartmental analysis to derive tmax, Cmax, AUC(0-24) on day 1. Peak and trough concentrations are summarized from days 2 through 7 to estimate drug accumulation and attainment of steady state.

### Lymphocyte assessment

Blood samples for absolute lymphocyte counts is collected at screening, at initial baseline (Day -2), Day 1 (6h postdose), Day 3 (predose), Day 5 (predose) and Day 7 (predose).

The samples are analyzed for pharmacodynamics.

Above procedure may be repeated and the patients are then treated Day 5 and followings with a daily maintenance dose of 0.5mg/kg. The patients have lower steady-state blood levels.

Above procedure may be repeated with following loading treatments:
1.
   Day 1: Subjects receive a single 2.5 mg FTY720 oral dose at the exact time the Day -1 dose was administered.
   Days 2-4: Subject receive a single 5 mg FTY720 oral dose on Day 2, a single 7.5 mg FTY720 oral dose on Day 3, and a single 10 mg FTY720 oral dose on Day 4, in order to achieve the FTY720 steady-state concentration typically measured in patients on chronic dosing of FTY720 2.5 mg qd.
   Day 5-7 and following: Subjects receive single 2.5 mg FTY720 oral doses once daily.
2.
   Day 1: Subjects receive a single 1.25 mg FTY720 oral dose at the exact time the Day - 1 dose was administered.
   Days 2-4: Subject receive a single 2.5 mg FTY720 oral dose on Day 2, a single 3.75 mg FTY720 oral dose on Day 3, and a single 5 mg FTY720 oral dose on Day 4, in order to achieve the FTY720 steady-state concentration typically measured in patients on chronic dosing of FTY720 1.25 mg qd.
   Day 5-7 and following: Subjects receive single 1.25 mg FTY720 oral doses once daily.

## Claims

1. A S1P receptor modulator or agonist for use as a medication for inhibition of graft rejection or treatment of an autoimmune disease or disorder, wherein the daily dosage of said S1P receptor modulator or agonist is increased stepwise from 4- to 12-fold the standard daily dosage of said S1P receptor modulator or agonist during the initial 3 to 6 days of treatment, such that a steady-state of the S1P receptor modulator or agonist blood levels is attained in less than a week, and wherein the standard daily dosage is the dosage necessary for a stable blood level concentration of the medication providing effective treatment.

2. A S1P receptor modulator or agonist for use in a method for inhibition of graft rejection or treatment of an autoimmune disease or disorder, wherein said S1P receptor modulator or agonist is formulated such that it can be administered to a subject by stepwise increasing the dosage of said S1P receptor modulator or agonist from 4- to 12-fold the standard daily dosage during the initial 3 to 6 days of treatment, such that a steady-state of the S1P receptor modulator or agonist blood levels is attained in less than a week, and wherein the standard daily dosage is the dosage necessary for a stable blood level concentration of the medication providing effective treatment.

3. The S1P receptor modulator or agonist according to claim 1 or 2, wherein the treatment is continued at a dosage lower than the standard daily dosage.

4. The S1P receptor modulator or agonist according to claim 1, 2 or 3, whereby the initial period is 4 or 5 days.

5. The S1P receptor modulator or agonist according to any preceding claim, whereby the stepwise increasing is of 10-fold.

6. The S1P receptor modulator or agonist according to any preceding claim, whereby the stepwise increasing is of 4-fold.

7. The S1P receptor modulator or agonist according to any of the preceding claims for treating multiple sclerosis, lupus nephritis, rheumatoid arthritis, inflammatory bowel diseases or psoriasis in a subject in need thereof.

8. A S1P receptor modulator or agonist for use in a method for treating a patient in need thereof, whereby said method comprises administering said S1P receptor modulator or agonist to a subject in such a way that the daily dosage of said S1P receptor modulator or agonist during the initial 3 to 6 days of treatment is increased stepwise from 4- to 12-fold the standard daily dosage of said S1P receptor modulator or agonist such that a steady-state of the S1P receptor modulator or agonist blood levels is attained in less than a week, and wherein the standard daily dose is the dosage necessary for a stable blood level concentration of the medication providing effective treatment.

9. A kit containing daily units of medication of an S1P receptor modulator or agonist of varying daily dosage, whereby the daily dosage of S1P receptor modulator or agonist is %; ½; and ¾ of the highest instalment dose of the S1P receptor modulator or agonist; and 4x the maintenance dose of the S1P receptor modulator or agonist, respectively, and units for the standard daily dosage of the S1P receptor modulator or agonist, or for the subsequent treatment with a daily dosage lower than the standard daily dosage, and wherein the standard daily dosage is the dosage necessary for a stable blood level concentration of the medication providing effective treatment.

10. A S1P receptor modulator or agonist according to any of claims 1 to 8 and the kit according to claim 9, wherein the S1P receptor modulator or agonist is 1-{4-[1-(4-cyclohexyl-3-trifluoromethyl-benzyloxyimino)-ethyl]-2-ethyl-benzyl}-azetidine-3-carboxylic acid, in free form or in a pharmaceutically acceptable salt form.

11. A S1P receptor modulator or agonist according to any of claims 1 to 8 and the kit according to claim 9, wherein the S1P receptor modulator or agonist is 1-{4-[1-(4-cyclohexyl-3-trifluoromethyl-benzyloxyimino)-ethyl]-2-ethyl-benzyl}-azetidine-3-carboxylic acid, in free form or in a pharmaceutically acceptable salt form, or a prodrug thereof.
